# EUROPEAN PATENT APPLICATION

(11) **EP 3 977 865 A1**
(43) Date of publication of application: **06.04.2022**
(21) Application number: 20815678.6
(22) Date of filing: 26.05.2020
(51) Int. Cl.: A23L 5/00, A23L 29/256, A23L 33/105

(54) **SODIUM EXCRETION PARTICLES**

(30) Priority: 28.05.2019 JP 2019099141
(71) Applicant: Toymedical Co., Ltd., Kumamoto 861-3131 (JP)
(72) Inventor: TAKESHITA, Hidenori, Kamimashikigun, Kumamoto 8613131 (JP); MAEDA, Ryoko, Kamimashikigun, Kumamoto 8613131 (JP)
(74) Representative: Dennemeyer & Associates S.A.
(86) International application number: PCT/JP2020/020791
(87) International publication number: WO 2020/241650

(57) **Abstract**

Provided is a new composition containing, as an active ingredient, alginate represented by ammonium alginate.

A food alginate-encapsulated microcapsule encapsulates alginate (excluding sodium salt) as an active ingredient. The microcapsule encapsulates alginate such that alginate does not directly contact a tongue when the microcapsule is adhered or added to food. Thus, the food can be taken with no feeling of unique smell and flavor of alginate, specifically ammonium alginate. Direct salt adsorption due to alginate is prevented in the food so that the food can be taken without impairing salty taste. In addition, the microcapsule disintegrates in a gastrointestinal tract, and alginate exercises a sodium adsorption ability. Accordingly, sodium excretion is promoted.

## Description

### TECHNICAL FIELD

The present invention relates to a sodium excretion particle. More specifically, the present invention relates to a sodium excretion particle used mixed with or adhered to food or a meal (cooked food) to adsorb sodium in a gastrointestinal tract after the food or the meal has been taken into a body, thereby promoting sodium excretion to the outside of the body.

### BACKGROUND ART

In addition to a dialysis, extremely-strict dietary limitations are on most of patients with impaired renal functions. Specifically, excess salt intake provides extremely adverse effect on health, and for this reason, a meal is basically provided with no/reduced salt. As a result, a patient's craving for food with a strong flavor is beyond normal individual's imagination.

With advancement of medical treatment, the risk of development of vascular diseases due to the hypertension, such as the stroke and cardiac infarction has become apparent. Accordingly, a blood pressure value as a normal value has decreased with the times. Thus, daily habits for avoiding the hypertension have attracted interest, and it has been recognized that as one of the daily habits, it is important to reduce sodium intake (reduce salt).

Because of such a situation, a technique relating to a composition for promoting salt excretion to the outside of a body has been disclosed (Patent Literature 1).

### CITATION LIST

### PATENT LITERATURE

PATENT LITERATURE 1: Japanese Patent No. 6497764

### SUMMARY OF INVENTION

### PROBLEMS TO BE SOLVED BY INVENTION

Patent Literature 1 discloses a technique provided by the inventor(s) of the present application, and is a technique relating to a food composition containing, as an active ingredient, ammonium alginate for promoting sodium excretion to the outside of the body.

Such a food composition is preferably used as a capsule encapsulating a mixture of ammonium alginate as an active ingredient and calcium alginate. With such a capsule, ammonium alginate adsorbs sodium in a gastrointestinal tract and is directly excreted to the outside of the body. Thus, sodium absorption is inhibited.

As described above, ammonium alginate has an excellent sodium excretion ability, but has a few problems in a case where ammonium alginate is used for food.

That is, ammonium alginate has unique smell and flavor. For this reason, in a case where ammonium alginate is used mixed with or adhered to food, the taste and flavor of the food might be impaired. In addition, when ammonium alginate is directly mixed with the food, ammonium alginate adsorbs salt contained in the food, and for this reason, the salty taste of the food might be impaired.

In view of the above-described situation, it is intended to develop a new composition containing, as an active ingredient, alginate represented by ammonium alginate.

### SOLUTION TO PROBLEMS

As a result of intensity study conducted by the inventor(s), the inventor(s) has arrived at formation of ammonium alginate into microparticles and formation of these microparticles into microcapsules, and has achieved the invention.

That is, the microcapsule encapsulating, as an active ingredient, ammonium alginate is formed so that food can be taken with no feeling of the taste of ammonium alginate. After the food has been taken, the microcapsule disintegrates in a gastrointestinal tract to exercise a sodium excretion ability.

The present invention has the following configuration.

A first configuration of the present invention is a food alginate-encapsulated microcapsule including alginate (excluding sodium salt) as an active ingredient.

A second configuration of the present invention is the food alginate-encapsulated microcapsule according to the first configuration, in which the alginate includes at least ammonium alginate.

A third configuration of the present invention is the food alginate-encapsulated microcapsule according to the first or second configuration, in which the particle size of the microcapsule is 10 to 3,000 µm.

A fourth configuration of the present invention is the food alginate-encapsulated microcapsule according to any one of the first to third configurations, in which the microcapsule is coated with any one or more of hydrosoluble or enteric polymer, copolymer, fat, sugars, sugar alcohol, or resin.

A fifth configuration of the present invention is a sodium excretion supplement including the food alginate-encapsulated microcapsule according to any one of the first to fourth configurations.

A sixth configuration of the present invention is the sodium excretion supplement according to the fifth configuration, in which the sodium excretion supplement is a noodle, a processed meat product, a paste product, a bread, and a seasoning agent.

A seventh configuration of the present invention is a sodium-containing seasoning agent including the food alginate-encapsulated microcapsule according to any one of the first to fourth configurations.

An eighth configuration of the present invention is a use method for promoting sodium excretion when food is taken, the use method including adding, upon use, the food alginate-encapsulated microcapsule according to any one of the first to fourth configurations to the food.

A ninth configuration of the present invention is a use method for promoting sodium excretion when food is taken, the use method including adhering, upon use, the food alginate-encapsulated microcapsule according to any one of the first to fourth configurations to the food.

### EFFECTS OF INVENTION

According to the present invention, a new composition containing, as an active ingredient, alginate represented by ammonium alginate can be provided.

That is, the food alginate-encapsulated microcapsule of the present invention is contained in food or adhered to a food surface, thereby providing the effect of inhibiting salt absorption in a body and promoting salt excretion to the outside of the body.

### BRIEF DESCRIPTION OF DRAWINGS

Fig. 1 shows a schematic view of a food alginate-encapsulated microcapsule.
Fig. 2 shows appearances of experimental examples of the food alginate-encapsulated microcapsule.
Fig. 3 shows appearance comparison among the food alginate-encapsulated microcapsule and salt.
Fig. 4 shows, in closeup, the food alginate-encapsulated microcapsule.
Fig. 5 shows, in closeup, the food alginate-encapsulated microcapsule.

### DESCRIPTION OF EMBODIMENTS

A food additive alginate-encapsulated microcapsule of the present invention will be described.

The food alginate-encapsulated microcapsule of the present invention is characterized in that alginate (excluding sodium salt) as an active ingredient is encapsulated in the food alginate-encapsulated microcapsule.

That is, when adhered or added to food, the microcapsule encapsulating alginate is used such that alginate does not directly contact a tongue. Thus, such food can be taken with no feeling of unique smell and flavor of alginate, specifically ammonium alginate. Direct salt adsorption due to alginate is prevented in the food so that the food can be taken without impairing salty taste. In addition, the microcapsule disintegrates in a gastrointestinal tract, and alginate exercises a sodium adsorption ability. Accordingly, sodium excretion is promoted.

As described above, the alginate-encapsulated microcapsule of the present invention provides a use method in which the alginate-encapsulated microcapsule is contained in food or adhered to a food surface to inhibit sodium absorption in a body and promote sodium excretion to the outside of the body.

Alginate is a compound represented by (C₆H₈O₆)ₙ, and is not specifically limited as long as alginate is used as the active ingredient encapsulated in the capsule.

That is, naturally-derived substances contained in algae or the like or chemically-synthesized substances may be used. As alginate, those with partially-substituted or modified chemical structures may be used without departing from the gist, which is sodium adsorption, of the present invention.

Alginate is not specifically limited as long as alginate fulfills the function of excreting sodium, and those with various molecular weights can be used.

Alginate to be typically used may include those with viscosity-average molecular weights of 100 to 10,000,000, more preferably 1,000 to 9,000,000, much more preferably 10,000 to 9,000,000, still much more preferably 100,000 to 9,000,000, and most preferably 100,000 to 8,000,000.

Alginate is not specifically limited as long as the microcapsule can be formed, and one in a wet state or one in a dry state may be used.

That is, at a microcapsule manufacturing step, alginate in the wet state may be coated and formed into a final product, alginate in the wet state may be dried after coating and formed into a final product, or alginate which is already in the dry state may be coated and formed into a final product. These alginates can be selected and used as necessary.

Considering viscosity, various alginates can be used. That is, alginates themselves do not normally have the same molecular weight, and for this reason, when used as a raw material, are specified according to the viscosity.

Alginate to be typically used may include those with viscosities of 10 to 1000 mPa·s at a 1% (w/v) concentration at 20°C or those with viscosities of 10 to 3000 mPa·s at a 10% (w/v) concentration at 20°C, more preferably viscosities of 20 to 900 mPa·s at the 1% (w/v) concentration at 20°C, much more preferably viscosities of 100 to 900 mPa·s, still much more preferably viscosities of 100 to 400 mPa·s, and most preferably viscosities of 300 to 400 mPa·s.

Alginate is not specifically limited as long as alginate is not sodium salt, and various salts can be used. Examples of these salts include organic cation salt, calcium salt, magnesium salt, iron salt, and zinc salt.

Note that sodium salt is excluded from alginate in the present invention, but it is not intended to exclude mixing of a slight amount of sodium salt from a technical limitation. That is, it is not intended to exclude, as alginate, even a slight amount of sodium salt in a case where alginate sodium is used as a raw material to chemically manufacture alginate by substitution reaction of salt.

Organic cation salt is preferably used as alginate. This can inhibit release of metal salt from the active ingredient, and therefore, the effect of safely applying such alginate to, e.g., a patient having an impaired renal function is provided.

Organic cation salt is not specifically limited as long as alginate can be formed and safety can be ensured, and various organic cations can be used.

Organic cation salt is preferably ammonium salt (ammonium alginate).

This can provide the effect of exerting a much better food composition sodium excretion ability in the present invention as compared to, e.g., other alginates. In addition, ammonium alginate in the wet state has a not-too-high moderate viscosity, and ammonium alginate in the dry state allows relatively-easy microencapsulation by particle size adjustment. Thus, ammonium alginate provides the effect of exerting excellent handleability and formability.

In the case of using ammonium alginate, those with viscosities of 20 to 900 mPa·s at the 1% (w/v) concentration at 20°C may be typically used. Moreover, ammonium alginate to be used may be more preferably those with viscosities of 100 to 400 mPa·s at the 1% (w/v) concentration at 20°C, and much more preferably viscosities of 300 to 400 mPa·s.

This can provide the effect of further improving the handleability and formability of ammonium alginate and more easily forming the capsule in the present invention.

In the present invention, alginate inorganic cation (alginate metal salt; excluding sodium) can be contained in addition to alginate organic cation.

This can provide the effect of easily holding the sodium adsorption ability while combining alginate organic cation and alginate inorganic cation with different viscosity properties to provide viscosity properties corresponding to a food composition and improving the handleability and formability of the food composition in the present invention.

Depending on the form of the food composition, a ratio between alginate organic cation and alginate inorganic cation varies. The weight ratio (Alginate Inorganic Cation Weight/Alginate Organic Cation Weight) of alginate inorganic cation to alginate organic cation may be typically 0.05 to 10, more preferably 0.1 to 10, and much more preferably 0.1 to 1.0.

Alginate inorganic cation to be used is not specifically limited as long as alginate inorganic cation is not sodium salt, and various metal salts can be used. Typically, one type or two or more types of potassium salt, calcium salt, magnesium salt, iron salt, zinc salt and the like can be selected.

Potassium salt is preferably excluded from alginate inorganic cation. This can provide the effect of preventing sodium release after the food composition of the present invention has been taken and safely applying such alginate inorganic cation to, e.g., a patient having an impaired renal function.

Polyvalent cation salt is preferably employed as alginate inorganic cation. This can maintain the sodium excretion ability with the content of metal ions in alginate being suppressed low, and can provide the effect of reducing metal release after the food composition of the present invention has been taken to safely applying such alginate inorganic cation to, e.g., a patient having an impaired renal function or a normal individual with the risk of developing hypertension.

Further, calcium salt is preferably used as alginate inorganic cation. Calcium alginate has a particularly low viscosity, and therefore, is combined with organic cation salt as described above to provide the effect of forming an optimal capsule while improving the formability of the food composition and holding the sodium adsorption ability.

Of the food alginate-encapsulated microcapsule of the present invention, "micro" is defined as a sufficiently-small additive used for food.

That is, the food alginate-encapsulated microcapsule of the present invention is based on the assumption that the food alginate-encapsulated microcapsule is, upon use, adhered or added to food. Thus, the food alginate-encapsulated microcapsule needs to have such a size that the taste and texture of food are not impaired upon use. Such a size varies depending on food for which the food alginate-encapsulated microcapsule is to be used, and for this reason, cannot be precisely defined.

A microcapsule can be used as the food alginate-encapsulated microcapsule, and the particle size thereof may be adjusted to several µm to several hundreds of µm or several µm to several thousands of µm. The particle size of such a microcapsule is, for example, 10 to 3000 µm, preferably 10 to 2500 µm, more preferably 20 to 2500 µm, much more preferably 30 to 2500 µm, and most preferably 40 to 2000 µm.

In a case where the food alginate-encapsulated microcapsule is the microcapsule (hereinafter merely referred to as an "alginate-encapsulated microcapsule"), alginate may be used as a core substance, and an edible coating material (hereinafter merely referred to as a "coating material") may be used as a capsule wall.

The coating material is not specifically limited as long as the effectiveness of alginate is not impaired and alginate can be released in the gastrointestinal tract, and can be selected from various points of view. As such a coating material, hydrosoluble or enteric polymer, copolymer, fat, sugars, sugar alcohol, or resin may be used. These materials can be used alone, or two or more types of these materials may be used in combination. Examples of a specific compound of the coating material as described above may include ethylene-vinyl acetate copolymer, ethyl acrylate-methacrylic acid copolymer, aminoalkyl methacrylate copolymer, polyethylene, polyamide, ethyl cellulose, ethyl cellulose water dispersion, polymethylmethacrylate, ethyl acrylate-methyl methacrylate copolymer dispersion, acetyl glycerin fatty acid ester, aminoalkyl methacrylate copolymer E, aminoalkyl methacrylate copolymer RS, gum arabic, powdered acacia, octyl-decyl triglyceride, Opadry AMB, Opadry OY-6950, Opadry OY-S-7135, Opadry OY-S-8471, Opadry OY-S-9607, Opadry OY-S-22829, Opadry OY-S-22835, Opadry OY-S-22961, olive oil, kaolin, cacao butter, prunella spike, castor wax, caramel, carnauba wax, carboxyvinyl polymer, carboxy methyl ethyl cellulose, carboxymethyl starch sodium, carmellose calcium, carmellose sodium, hydrated silicon dioxide, dried aluminium hydroxide gel, dried milky white lac, dried methacrylate copolymer LD, kanbai powder, fish scale powder, gold foil, silver foil, triethyl citrate, glycerin, glycerin fatty acid ester, magnesium silicate, light anhydrous silicic acid, light anhydrous silicic acid-containing hydroxypropylcellulose, light liquid paraffin, whale wax, crystalline cellulose, hardened oil, synthetic aluminum silicate, synthetic wax, high glucose molasses, hard wax, succinylated gelatin, wheat flour, wheat starch, rice starch, cellulose acetate, vinyl acetate resin, cellulose acetate phthalate, saran beeswax white beeswax, titanium oxide, magnesium oxide, methylmetharylate, dimethylamino ethyl methacrylate-methyl methacrylate copolymer, dimethylpolysiloxane (for internal use), dimethylpolysiloxane-silicon dioxide mixture, burnt gypsum, sucrose fatty acid ester, jinko powder, aluminum hydroxide gel, hydrogenated rosin glycerin ester, stearyl alcohol, cetostearyl alcohol, stearic acid, aluminum stearate, calcium stearate, polyoxyl stearate 40, magnesium stearate, purified gelatin, purified shellac, purified white sugar, zeine, sorbitan sesquioleate, cetanol, gypsum, gelatin, shellac, sorbitan fatty acid ester, D-sorbitol, D-sorbitol solution, tricalcium phosphate, calcium carbonate, magnesium carbonate, simple syrup, burnt silver foil, precipitated calcium carbonate, low substituted hydroxypropylcellulose, turpentine resin, starch (soluble), corn syrup, corn oil, triacetin, calcium lactate, lactose, concentrated glycerin, white shellac, white sugar, honey, hard fat, paraffin, pearl powder, white potato starch, hydroxypropylcellulose, hydroxypropylmethylcellulose phthalate, piperonyl butoxide, castor oil, diethyl phthalate, dibutyl phthalate, butylphthalylbutyl glycolate, glucose, fumaric acid-stearic acid-polyvinyl acetal diethylamino acetate-hydroxypropylcellulose 2910 mixture, pullulan, propylene glycol, bentonite, povidone, polyoxyethylene hardened castor oil 40, polyoxyethylene hardened castor oil 60, polyoxyethylene (105) polyoxypropylene (5) glycol, polyoxyethylene (160) polyoxypropylene (30) glycol, polysorbate 80, polyvinyl acetal diethylaminoacetate, D-mannitol, molasses, beeswax, myristyl alcohol, anhydrous silicic acid hydrate, anhydrous phthalic acid, anhydrous calcium hydrogen phosphate, methacrylate copolymer L, methacrylate copolymer LD, methacrylate copolymer S, magnesium aluminometasilicate, methylcellulose, methyl acrylate, 2-methyl-5-vinylpyridinemethylacrylate-methacrylic acid copolymer, wood wax, aluminum monostearate, glycerin monostearate, sorbitan monolaurylate, montanic acid ester wax, medical charcoal, lauromacrogol, calcium sulfate, liquid paraffin, DL-malic acid, calcium monohydrogen phosphate, calcium hydrogen phosphate, sodium hydrogen phosphate, calcium dihydrogen phosphate, rosin, hydroxypropyl cellulose (HPC), Hypromellose (HPMC), carboxyvinyl polymer, cross-linked polyhydroxyethyl methacrylate, polyvinyl alcohol, polysaccharides (starch, dextran, alginic acid, chitosan), albumin, fibrinogen, collagen, gelatin, polylactic acid-glycolic acid copolymer, and polyorthoester. These compounds can be used alone, or two or more types of these compounds can be used in combination.

The method for manufacturing the alginate-encapsulated microcapsule is not specifically limited as long as such manufacturing method is implementable, and various manufacturing methods can be used. That is, depending on the types of core substance and coating material, a chemical manufacturing method, a physicochemical manufacturing method, a mechanical manufacturing method and the like can be selected and used as necessary.

The chemical manufacturing method is a method in which the capsule wall is formed using chemical reaction to produce the microcapsule, and examples thereof include an interfacial polymerization method and an in-site polymerization method.

The physicochemical manufacturing method is a method in which the microcapsule is produced not by chemical reaction but by solidification, deposition or the like, and examples thereof include a liquid drying method and a coacervation method.

The mechanical manufacturing method is a method in which the microcapsule is produced using a machine, and examples thereof include a spray drying method and a dry blending method.

The flow of manufacturing the alginate-encapsulated microcapsule will be described.

First, alginate with a predetermined average particle size is used as the core substance. In this case, the average particle size of alginate can be adjusted as necessary according to intended use. For example, in a case where the alginate-encapsulated microcapsule is contained as a seasoning agent, the average particle size of alginate as the core substance may be adjusted with reference to 500 µm or less. The average particle size may be adjusted using a mesh filter. Selection of alginate and adjustment of the average particle size as described above can be performed as common knowledge in the art.

Considering handleability and economy, alginate with one ingredient is preferably used. Moreover, alginate is preferably used as alginate.

After the core substance has been selected, the coating material for coating the core substance is selected.

The coating material can be selected as necessary according to a coating method and intended use. For example, when the coating material is sprayed for coating, the coating material suitable for spraying can be selected. As such a coating material, HPMC, zein, shellac, and the like can be used.

The method for spraying the coating material to coat the core substance can be performed using normal equipment used by those skilled in the art.

The food alginate-encapsulated microcapsule can be, upon use, adhered or added to food. Preferably, the alginate-encapsulated microcapsule is added/mixed as one food material, and as necessary, is subject to treatment such as thermal treatment. When such food is taken, alginate does not directly contact the tongue. Thus, the food can be taken with no feeling of unique smell and flavor of alginate, specifically ammonium alginate. Moreover, direct salt adsorption due to alginate is prevented in the food, and therefore, the food can be taken without impairing salty taste. In addition, the microcapsule disintegrates in the gastrointestinal tract, and alginate exercises the sodium adsorption ability. Accordingly, sodium excretion is promoted.

The food alginate-encapsulated microcapsule is not specifically limited as long as such a microcapsule fulfills the above-described role, and upon use, can be added to various types of food. Such food is provided as a sodium excretion supplement, and examples thereof include noodles, processed meat products, paste products, breads, and seasoning agents.

The food alginate-encapsulated microcapsule can be preferably used in such a form that the microcapsule is contained in a seasoning agent in a powder form. That is, a predetermined amount of food alginate-encapsulated microcapsule is contained in a seasoning agent containing sodium. Upon use, a food surface can be coated with such a powder seasoning agent containing the food alginate-encapsulated microcapsule. Moreover, the powder seasoning agent can be used as a seasoning agent inhibiting sodium absorption.

The powder seasoning agent does not necessarily contain sodium. That is, when the seasoning agent itself does not contain sodium, if food itself contains salt, the powder seasoning agent containing the food alginate-encapsulated microcapsule is used for coating so that sodium absorption can be inhibited.

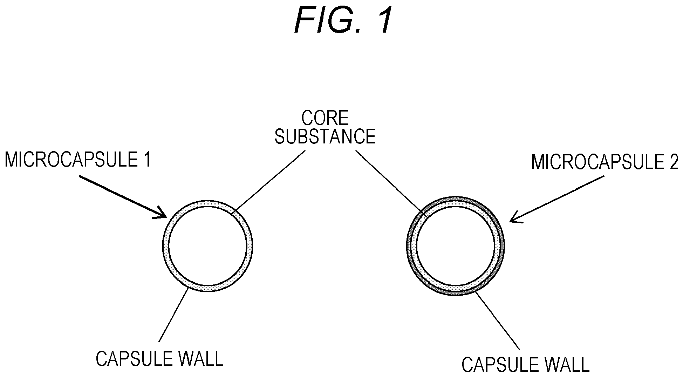

Fig. 1 shows a schematic view of the food alginate-encapsulated microcapsule.

As shown in Fig. 1, the core substance is encapsulated in the capsule wall. Alginate is encapsulated as the core substance in this configuration. Alginate may be used alone as the core substance, or a combination of multiple types of alginate or a combination of alginate with other substances may be used as the core substance. The same also applies to the capsule wall, and a single compound or a combination of multiple types of compounds may be used to form the capsule wall. Moreover, the capsule wall may be a single-layer capsule wall as shown on the left side in Fig. 1, or may be a multilayer capsule wall as shown on the right side in Fig. 1.

### Examples

The alginate-encapsulated microcapsule of the present invention will be described in detail.

### <<I. Experiment Method>>

Using ammonium alginate as a raw material, samples as shown in a table were produced. Note that the summary of manufacturing methods in the table is as follows.

### <Manufacturing Method 1. Spray Dry Method>

[Device Used] Nitrogen Gas Sealed Circulation Type Spray Dryer (Ohkawara Kakohki Co., Ltd, Circlex CL-8i)
[Summary] HPMC was sprayed onto ammonium alginate to produce powder with a 9% (w/w) coating. The average particle size was adjusted to about 60 µm.

### <Manufacturing Method 2. Fluidized Bed Granulation Coating>

[Device Used] Fluidized Bed Granulation Coating Device (Freund Corporation, Flowcoater FL-LABO)
[Summary] Various coating substances were sprayed onto ammonium alginate to produce coated powder. The average particle size was adjusted to about 115 to 240 µm.

### <Manufacturing Method 3. Oscillating Fluidized Bed Granulation Coating>

[Device Used] Oscillating Fluidized Bed Granulation Coating Machine (Powrex Corporation, FD-MP-01D)
[Summary] Various coating substances were sprayed onto ammonium alginate to produce coated powder. The average particle size was adjusted to about 80 to 250 µm.

**[Table 1]**

| **Sample** | **Manufacturing Method** | **Coating Substance** | | | | **Average Particle Size (µm)** |
|---|---|---|---|---|---|---|
| **Experimental Example 1** | **1** | **HPMC** | **9.0%** | | | **60** |
| **Experimental Example 2** | **2** | **HPMC** | **10.0%** | | | **135** |
| **Experimental Example 3** | **2** | **HPMC** | **5.0%** | **shellac** | **5.0%** | **115** |
| **Experimental Example 4** | **2** | **zein** | **10.0%** | | | **130** |
| **Experimental Example 5** | **2** | **zein** | **5.5%** | **shellac** | **5.5%** | **115** |
| **Experimental Example 6** | **2** | **HPMC** | **30.0%** | | | **230** |
| **Experimental Example 7** | **2** | **HPMC** | **30.0%** | **shellac** | **8.0%** | **240** |
| **Experimental Example 8** | **2** | **zein** | **30.0%** | | | **210** |
| **Experimental Example 9** | **2** | **zein** | **30.0%** | **shellac** | **8.0%** | **230** |
| **Experimental Example 10** | **3** | **zein** | **10.0%** | | | **80-250** |
| **Experimental Example 11** | **3** | **zein** | **20.0%** | | | **80~250** |
| **Experimental Example 12** | **3** | **zein** | **30.0%** | | | **80~250** |
| **Experimental Example 13** | **3** | **zein** | **30.0%** | **shellac** | **10.0%** | **80~250** |

### <<II. Experimental Results>>

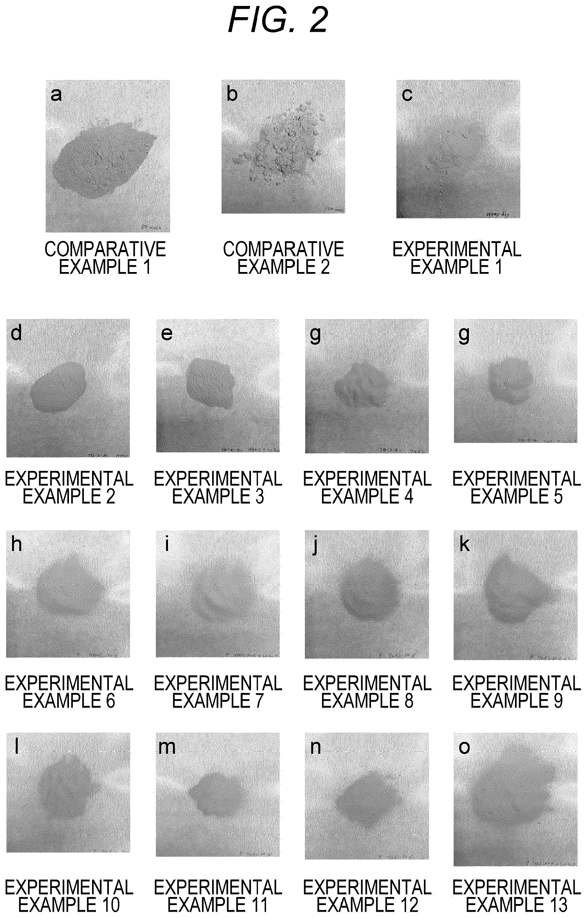

1. Fig. 2 shows the appearance of the produced powder. Note that comparative examples include Comparative Example 1 which is ammonium alginate powder processed with an 80 mesh and raw material powder for Experimental Examples 6 to 9 and Comparative Example 2 which is ammonium alginate powder processed with a 150 mesh and raw material powder for Experimental Examples 2 to 5 and Experimental Examples 10 to 13. Moreover, as raw material powder for Experimental Example 1, ammonium alginate powder (an average particle size of 10 µm, not shown) is used.
(1) Experimental Example 1 shows entirely-fine particles and a smooth powder form.
(2) Experimental Examples 2 to 9 also show entirely-fine particles and a smooth powder form.
(3) Experimental Examples 10 to 13 also show entirely-fine particles and a smooth powder form.
(4) No significant difference has been found among the manufacturing methods, and favorable appearance and properties were shown.
(5) In addition, there is a certain level of smell in the comparative examples, and on the other hand, such smell is reduced or eliminated in the experimental examples.

Specifically, Experimental Examples 6 to 8 and Experimental Examples 12 and 13 prominently show these effects.

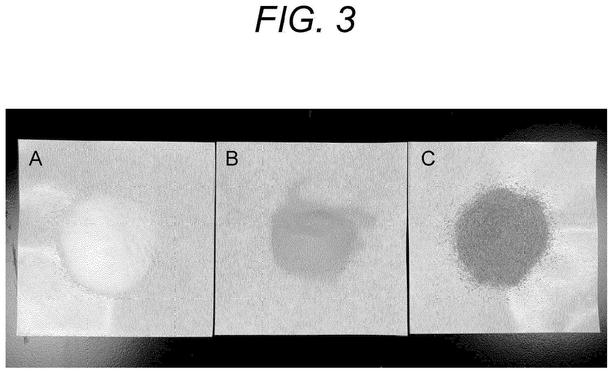

2. Fig. 3 shows photographs for comparing the produced powder (Experimental Example 9) and commercially-available salt and salt-and-pepper. In the figure, A indicates the salt, B indicates the powder of Experimental Example 9, and C indicates the salt-and-pepper. In any experimental example, the powder shows no significant difference in appearance from the commercially-available salt. This shows that there is no problem even if the produced powder is used with the produced powder being mixed with a general seasoning agent.

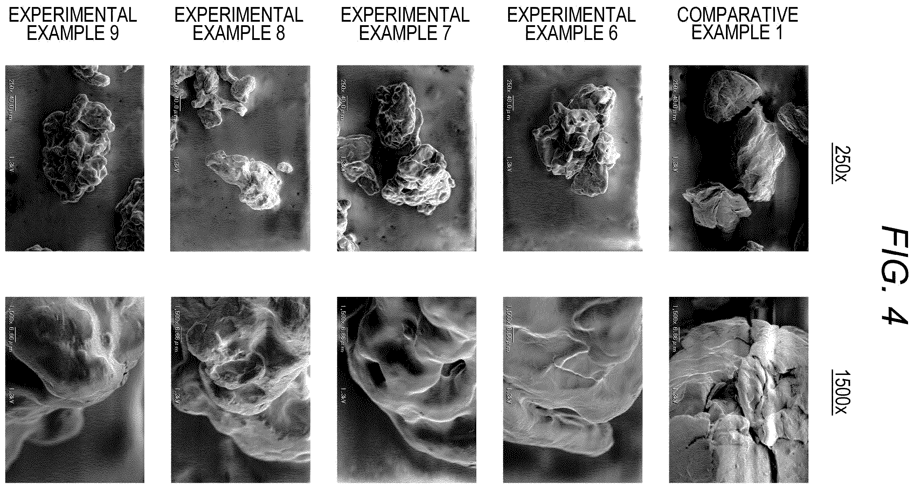

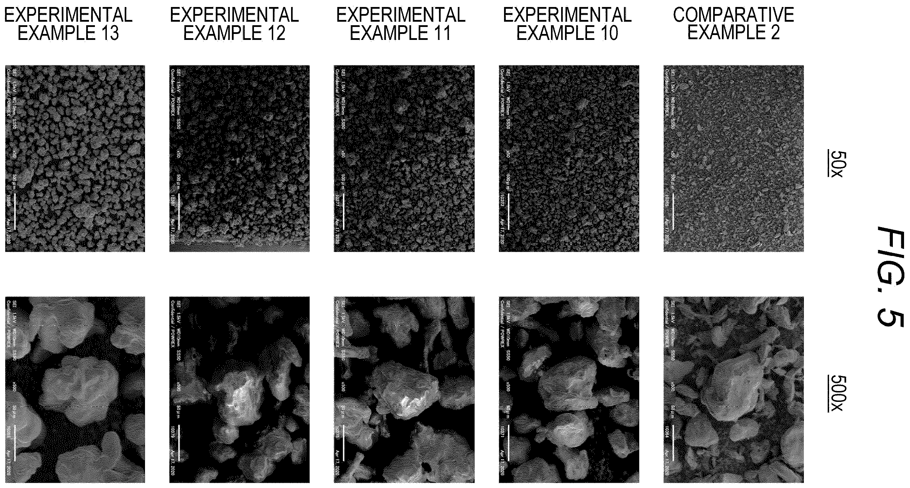

2. Observation results of each experimental example by an electron scanning microscope are shown in Figs. 4 and 5.
(1) Fig. 4 shows that as compared to the comparative example, the coating substance adheres to the surface and such a surface is smoothly coated in the experimental examples.
(2) Fig. 5 also shows that as compared to the comparative example, the coating substance adheres to the surface and such a surface is smoothly coated in the experimental examples.

3. Evaluation results of the sodium adsorption ability of each sample are shown in Tables 2 to 4. These experiments show the results obtained in such a manner that 50 mg of each sample was added to 1 mL of each test solution and a sodium concentration was measured over time.

(1) Experimental Example 1 shows a slightly-high salt concentration as a whole as compared to Comparative Example 1, but shows a decrease in the salt concentration over time.

**[Table 2]**

| **Sample** | **Test Solution (Salt Concentration 1%)** | **Salt Concentration Measurement Value (%)** | | |
|---|---|---|---|---|
| | | **1min** | **5min** | **10min** |
| **Comparative Example 1** | **1% NaCl, pH7.0** | **0.69** | **0.59** | **0.60** |
| | **1% NaCl, 0.05M PBS, pH6.8** | **0.67** | **0.61** | **0.60** |
| | **1% NaCl, pH3.0** | **0.69** | **0.68** | **0.65** |
| **Experimental Example 1** | **1% NaCl, pH7.0** | **0.71** | **0.67** | **0.62** |
| | **1% NaCl, 0.05M PBS, pH6.8** | **0.77** | **0.67** | **0.65** |
| | **1% NaCl, pH3.0** | **0.69** | **0.69** | **0.69** |

(2) Experimental Examples 2 to 9 show a slightly-high salt concentration as a whole as compared to Comparative Example 1, but shows a decrease in the salt concentration over time.

(3) Experimental Example 3 shows a salt concentration level similar to that of Comparative Example 1 and a decrease in the salt concentration over time.

**[Table 3]**

| **Sample** | **Test Solution (Salt Concentration 1%)** | **Salt Concentration Measurement Value(%)** | | |
|---|---|---|---|---|
| | | **1min** | **5min** | **10min** |
| **Comparative Example 1** | **1% NaCl, pH7.0** | **0.69** | **0.59** | **0.60** |
| | **1% NaCl, 0.05M PBS, pH6.8** | **0.67** | **0.61** | **0.60** |
| | **1% NaCl, pH3.0** | **0.69** | **0.68** | **0.65** |
| **Experimental Example 2** | **1% NaCl, pH7.0** | **0.84** | **0.71** | **0.70** |
| | **1% NaCl, 0.05M PBS, pH6.8** | **0.75** | **0.68** | **0.61** |
| | **1% NaCl, pH3.0** | **0.77** | **0.69** | **0.65** |
| **Experimental Example 3** | **1% NaCl, pH7.0** | **0.68** | **0.67** | **0.62** |
| | **1% NaCl, 0.05M PBS, pH6.8** | **0.66** | **0.64** | **0.64** |
| | **1% NaCl, pH3.0** | **0.68** | **0.68** | **0.68** |
| **Experimental Example 4** | **1% NaCl, pH7.0** | **0.77** | **0.68** | **0.60** |
| | **1% NaCl, 0.05M PBS, pH6.8** | **0.72** | **0.66** | **0.63** |
| | **1% NaCl, pH3.0** | **0.94** | **0.83** | **0.72** |
| **Experimental Example 5** | **1% NaCl, pH7.0** | **0.78** | **0.66** | **0.65** |
| | **1% NaCl, 0.05M PBS, pH6.8** | **0.71** | **0.65** | **0.57** |
| | **1% NaCl, pH3.0** | **0.87** | **0.84** | **0.74** |
| **Experimental Example 6** | **1% NaCl, pH7.0** | **0.79** | **0.80** | **0.76** |
| | **1% NaCl, 0.05M PBS, pH6.8** | **0.86** | **0.85** | **0.84** |
| | **1% NaCl, pH3.0** | **0.78** | **0.71** | **0.73** |
| **Experimental Example 7** | **1% NaCl, pH7.0** | **0.92** | **0.84** | **0.81** |
| | **1% NaCl, 0.05M PBS, pH6.8** | **0.93** | **0.87** | **0.84** |
| | **1% NaCl, pH3.0** | **0.89** | **0.90** | **0.76** |
| **Experimental Example 8** | **1% NaCl, pH7.0** | **0.86** | **0.81** | **0.81** |
| | **1% NaCl, 0.05M PBS, pH6.8** | **0.94** | **0.89** | **0.89** |
| | **1% NaCl, pH3.0** | **0.98** | **0.77** | **0.76** |
| **Experimental Example 9** | **1% NaCl, pH7.0** | **0.98** | **0.87** | **0.83** |
| | **1% NaCl, 0.05M PBS, pH6.8** | **0.88** | **0.88** | **0.87** |
| | **1% NaCl, pH3.0** | **0.78** | **0.79** | **0.77** |

(4) Experimental Examples 10 to 13 show a slightly-high salt concentration as a whole as compared to Comparative Example 1, but shows a decrease in the salt concentration over time.

**[Table 4]**

| **Sample** | **Test Solution (Salt Concentration 1%)** | **Salt Concentration Measurement Value(%)** | | |
|---|---|---|---|---|
| | | **1min** | **5min** | **10min** |
| **Comparative Example 1** | **1% NaCl, pH7.0** | **0.69** | **0.59** | **0.60** |
| | **1% NaCl, 0.05M PBS, pH6.8** | **0.67** | **0.61** | **0.60** |
| | **1% NaCl, pH3.0** | **0.69** | **0.68** | **0.65** |
| **Experimental Example 10** | **1% NaCl, pH7.0** | **0.80** | **0.72** | **0.71** |
| | **1% NaCl, 0.05M PBS, pH6.8** | **0.80** | **0.75** | **0.72** |
| | **1% NaCl, pH3.0** | **0.75** | **0.67** | **0.65** |
| **Experimental Example 11** | **1% NaCl, pH7.0** | **0.70** | **0.70** | **0.67** |
| | **1% NaCl, 0.05M PBS, pH6.8** | **0.76** | **0.79** | **0.78** |
| | **1% NaCl, pH3.0** | **0.75** | **0.75** | **0.76** |
| **Experimental Example 12** | **1% NaCl, pH7.0** | **0.85** | | **0.74** |
| | **1% NaCl, 0.05M PBS, pH6.8** | **0.85** | **0.77** | **0.77** |
| | **1% NaCl, pH3.0** | **0.83** | **0.77** | **0.72** |
| **Experimental Example 13** | **1% NaCl, pH7.0** | **0.88** | **0.72** | **0.74** |
| | **1% NaCl, 0.05M PBS, pH6.8** | **0.84** | **0.82** | **0.81** |
| | **1% NaCl, pH3.0** | **0.76** | **0.71** | **0.72** |

(5) From these results, it was assumed that the experimental examples show, as compared to the comparative example, the slightly-high salt concentration and the decrease in the salt concentration over time because of the effect of the coating of the ammonium alginate raw material powder.

(6) Such a tendency varies according to, e.g., the type and ratio of the coating. Thus, it was found that the tendency of salt adsorption can be controlled by adjustment of the coating.

4. Table 5 shows measurement results of the viscosity of each sample. The viscosity was measured using an oscillation viscometer (VISCOMATE, VM-10A) while 100 mL of each solvent to which 0.2 g of each sample was added is being stirred at 500 rpm. Note that in the table, Comparative Example 2 is raw material powder for Experimental Examples 2 to 5, and Comparative Example 3 is raw material powder for Experimental Example 1 and ammonium alginate powder with 10 µm.

(1) As compared to the comparative example, the tendency shows that the viscosity increases over time in the experimental examples. It was assumed that there is a difference in this tendency depending on the solvent and such a difference is caused due to the coating.

(2) Experimental Examples 1 to 3 using HPMC shows, at pH 6.8, a relatively-high viscosity after one minute as compared to Experimental Examples 4 to 5 using zein. This indicates that HPMC itself influences the viscosity.

**[Table 5]**

| **Sample** | **Solvent** | | **Viscosity (mPa·s)** | | |
|---|---|---|---|---|---|
| | | **After 1 min** | **After 5 min** | **After 10 min** | **After 30 min** |
| **Comparative Example 2** | **DW** | **3.81** | **7.07** | **8.01** | **8.51** |
| | **pH3.0** | **2.25** | **2.42** | **3.22** | **4.70** |
| | **pH6.8** | **3.22** | **3.31** | **2.32** | **5.12** |
| **Experimental Example 2** | **DW** | **10.30** | **9.24** | **9.38** | **8.40** |
| | **pH3.0** | **1.96** | **3.52** | **2.18** | **3.18** |
| | **pH6.8** | **2.44** | **2.58** | **3.75** | **3.15** |
| **Experimental Example 3** | **DW** | **9.30** | **8.50** | **8.03** | **7.97** |
| | **pH3.0** | **3.05** | **3.32** | **3.03** | **2.35** |
| | **pH6.8** | **3.24** | **2.58** | **1.83** | **3.10** |
| **Experimental Example 4** | **DW** | **3.58** | **6.72** | **7.64** | **6.96** |
| | **pH3.0** | **1.94** | **2.65** | **3.53** | **2.77** |
| | **pH6.8** | **2.30** | **3.16** | **2.68** | **2.86** |
| **Experimental Example 5** | **DW** | **8.96** | **8.72** | **11.00** | **7.36** |
| | **pH3.0** | **3.64** | **2.43** | **3.18** | **1.82** |
| | **pH6.8** | **2.00** | **3.82** | **2.62** | **2.80** |
| **Comparative Example 3** | **DW** | **4.87** | **7.53** | **8.94** | **10.50** |
| | **pH3.0** | **4.07** | **10.50** | **5.09** | **4.36** |
| | **H6.8** | **2.95** | **3.67** | **3.95** | **4.35** |
| **Experimental Example 1** | **DW** | **6.00** | **5.03** | **5.94** | **9.44** |
| | **pH3.0** | **2.00** | **3.08** | **2.49** | **4.07** |
| | **pH6.8** | **4.56** | **4.05** | **3.06** | **3.65** |

## Claims

1. A food alginate-encapsulated microcapsule comprising:
alginate, which excludes sodium salt, as an active ingredient.

2. The food alginate-encapsulated microcapsule according to claim 1, wherein the alginate includes at least ammonium alginate.

3. The food alginate-encapsulated microcapsule according to claim 1 or 2, wherein
a particle size of the microcapsule is 10 to 3,000 µm.

4. The food alginate-encapsulated microcapsule according to any one of claims 1 to 3, wherein
the microcapsule is coated with any one or more of hydrosoluble or enteric polymer, copolymer, fat, sugars, sugar alcohol, or resin.

5. A sodium excretion supplement comprising:
the food alginate-encapsulated microcapsule according to any one of claims 1 to 4.

6. The sodium excretion supplement according to claim 5, wherein
the sodium excretion supplement is a noodle, a processed meat product, a paste product, a bread, and a seasoning agent.

7. A sodium-containing seasoning agent comprising:
the food alginate-encapsulated microcapsule according to any one of claims 1 to 4.

8. A use method for promoting sodium excretion when food is taken, comprising:
adding, upon use, the food alginate-encapsulated microcapsule according to any one of claims 1 to 4 to the food.

9. A use method for promoting sodium excretion when food is taken, comprising:
adhering, upon use, the food alginate-encapsulated microcapsule according to any one of claims 1 to 4 to the food.
